Europäisches Patentamt

European Patent Office

Office européen des brevets

(19)

(11) Veröffentlichungsnummer: **0 318 647 B1**

(12) # EUROPÄISCHE PATENTSCHRIFT

(45) Veröffentlichungstag der Patentschrift: **27.12.91**

(51) Int. Cl.⁵: **A61F 9/02**

(21) Anmeldenummer: **88114498.4**

(22) Anmeldetag: **06.09.88**

(54) **Schutzbrille.**

(30) Priorität: **03.12.87 DE 3740949**

(43) Veröffentlichungstag der Anmeldung:
**07.06.89 Patentblatt 89/23**

(45) Bekanntmachung des Hinweises auf die
Patenterteilung:
**27.12.91 Patentblatt 91/52**

(84) Benannte Vertragsstaaten:
**DE FR GB**

(56) Entgegenhaltungen:
**GB-A- 1 092 829**
**US-A- 4 414 693**
**US-A- 4 698 957**

(73) Patentinhaber: **AUERGESELLSCHAFT GMBH**
**Thiemannstrasse 1**
**W-1000 Berlin 44(DE)**

(72) Erfinder: **Hünnebeck, Volker**
**Ostpreussendamm 105**
**W-1000 Berlin 45(DE)**

## Beschreibung

Die Erfindung betrifft eine Schutzbrille gegen Gase nach dem Oberbegriff des Anspruchs 1.

Eine derartige Schutzbrille ist aus der US-A-4 698 857 bekannt. Hierbei sind die jeweils ein Auge des Benutzers umschließenden Augenräume als ein Faltenbalg mit einer Vielzahl von Faltenteilen ausgebildet, wobei die Faltenteile zueinander symmetrisch am Brillenkörper angeordnet sind. Nachteilig an der bekannten Ausführung ist, daß durch das Vorsehen einer Vielzahl von symmetrisch ausgerichteter Faltenteile einerseits die zentrale Sichtöffnung für das Auge verhältnismäßig klein ausfällt und das Sichtfeld dadurch eingeschränkt ist und andererseits die Brille im verpackten Zustand einen verhältnismäßig großen Raum beansprucht.

Der Erfindung liegt deshalb die Aufgabe zugrunde, eine Schutzbrille der eingangs genannten Art zu schaffen, die ein enges Aufeinanderfalten und Zusammendrücken des Brillenkörpers und der Fassungen mit den Augengläsern zu einem flachen Päckchen ermöglicht und ein geringes Tragegewicht aufweist.

Diese Aufgabe wird erfindungsgemäß durch die kennzeichnenden Merkmale des Anspruchs 1 gelöst.

Die mit der Erfindung erzielten Vorteile bestehen insbesondere darin, daß durch das hohe Rückstellvermögen des mit den Faltenbälgen und den Fassungen für die Augengläser einstückig ausgebildeten Brillenkörpers, die Augengläser unter einer gewissen Vorspannung dicht in den Fassungen eingesetzt werden können, wodurch sonst übliche Spannschellen oder dgl. entfallen. Als ein weiterer Vorteil ist zu nennen, daß der Faltenbalg eine Art Pufferzone zwischen der formstabilen Fassung für die Gläser und dem sich an das Gesicht anpassenden Brillenkörper bildet, wodurch beim Aufsetzen des Brillenkörpers die Gläser fest in der Fassung verbleiben, ohne daß eine Schelle um die Fassung gelegt werden muß.

Die Erfindung wird nachstehend anhand eines Ausführungsbeispiels in der Zeichnung näher beschrieben. Es zeigt

Fig. 1    eine Voransicht der erfindungsgemäßen Schutzbrille

Fig. 2    eine Draufsicht der Schutzbrille

Fig. 3    eine vergrößerte Schnittdarstellung nach der Linie I-I in Fig. 1, und

Fig. 4    eine Schnittdarstellung nach Fig. 3, wobei die Fassungen mit dem Augenglas über den erfindungsgemäßen Faltenbalg eng am Brillenkörper angedrückt sind.

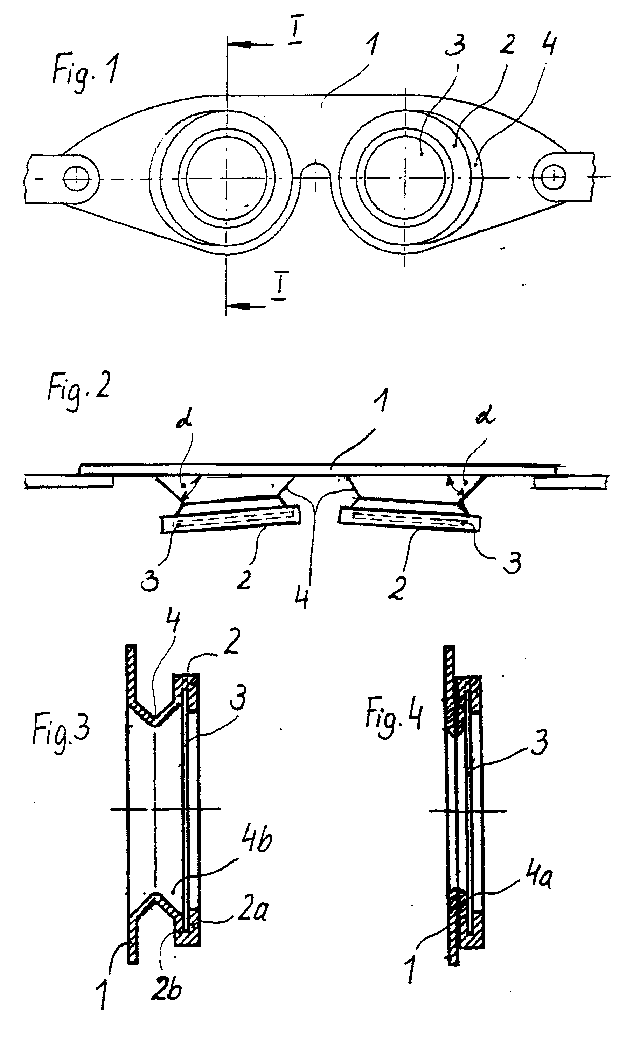

Wie die Figuren 1 und 2 zeigen, besteht die Schutzbrille im wesentlichen aus einem Brillenkörper 1, an dem jeweils ein von der ebenen Fläche des Brillenkörpers abstehender Faltenbalg 4, unter einem entsprechenden Winkel α asymmetrisch angeformt ist, und jeweils aus einer am Faltenbalg 4 ausgebildeten Fassung 2, in die ein Augenglas 3 unverlierbar dicht eingesetzt ist.

Wie Fig 3. zeigt, ist die Fassung 2 hierbei als ein die Augengläser 3 am Umfang umfassender Rahmen ausgebildet, der einen vorderen Fassungsrand 2a und einen hinteren Fassungsrand 2b aufweist. Die Fassungsränder 2a und 2b üben auf beiden Seiten des in die Fassung 2 eingesetzten Augenglases 3 eine so hohe Vorspannung aus, daß die Augengläser unverlierbar fest und gasdicht von dem Fassungsrahmen umfaßt werden. Der Brillenkörper 1 mit den Faltenbälgen 4 und den Fassungen 2 besteht aus einem Stück und ist aus einem elastischen Kunststoff hergestellt, und zwar vorzugsweise aus einem Silikon-Elastomere mit hoher Rückstellkraft. Der elastische Brillenkörper 1 paßt sich den unterschiedlichsten Gesichtsformen an.

Fig. 4 zeigt, wie der Faltenbalg 4 mit den Augengläsern 3 des Augenraumes flach zusammengefaltet ist, und zwar derart, daß ein Faltenteil 4a des Faltenbalgs großflächig am Augenglas 3 anliegt und dieses somit gegen ein Herausdrücken nach innen schützt.

Durch die Ausgestaltung des Augenraumes als elastischen Faltenbalg, dient die dem Augenglas 3 zugewandte Faltentasche 4b vorteilhaft als Raum zum Sammeln von Schwitz- und/oder Kondenswasser, so daß dieses mit der Haut des Benutzers nicht in Berührung kommt.

## Patentansprüche

1.  Schutzbrille gegen Gase, bestehend aus einem am Gesicht des Benutzers anliegenden Brillenkörper (1) und zwei, jeweils ein Auge des Benutzers umschließende Augenräume mit Fassung (2) zur Aufnahme von Augengläsern (3), wobei der Augenraum als ein Faltenbalg (4) ausgebildet ist, dadurch gekennzeichnet, daß

    a) der Faltenbalg (4) an der ebenen Fläche des Brillenkörpers (1) unter einen entsprechenden Winkel (α) asymmetrisch angeformt ist, und

    b) der Brillenkörper (1) mit den Faltenbälgen (4) und den Fassungen (2) für die Augengläser (3) als ein Stück ausgebildet ist, welches aus einem elastischen Kunststoff besteht, der ein hohes Rückstellvermögen aufweist.

2.  Schutzbrille nach Anspruch 1, dadurch gekennzeichnet, daß der Kunststoff der Schutzbrille vorzugsweise ein Silikon-Elastomere ist.

3. Schutzbrille nach den Ansprüchen 1 und 2, dadurch gekennzeichnet, daß die Fassungen (2) jeweils als ein die Augengläser (3) beidseitig umfassender Rahmen ausgebildet ist, der einen vorderen und einen hinteren Fassungsrand (2a, 2b) aufweist.

4. Schutzbrille nach den Ansprüchen 1 bis 3, dadurch gekennzeichnet, daß die Fassungsränder (2a, 2b) auf beiden Seiten der Augengläser (3) eine so hohe Vorspannung ausüben, daß die Augengläser in den Fassungen (2) unverlierbar fest und gasdicht angeordnet sind.

5. Schutzbrille nach den Ansprüchen 1 bis 4, dadurch gekennzeichnet, daß im zusammengefalteten Zustand des Faltenbalgs (4) ein Faltenteil (4a) großflächig am Augenglas (3) anliegt.

## Claims

1. Goggles for protection from gases, consisting of a goggles body (1) resting against the user's face, and two eye spaces, each surrounding one eye of the user, with a frame (2) to receive eye glasses (3), in which the eye space is constructed as a bellows (4), characterised in that
   a) the bellows (4) is formed asymmetrically on the flat surface of the goggles body (1) at a corresonding angle ($\alpha$), and
   b) the goggles body (1) is constructed as one piece with the bellows (4) and the frames (2) for the eye glasses (3), which piece consists of an elastic plastics material which has a high restoring capacity.

2. Goggles according to Claim 1, characterised in that the plastics material of the goggles is preferably a silicon elastomer.

3. Goggles according to Claims 1 and 2, characterised in that the frames (2) are in each case constructed as a framework surrounding the eye glasses (3) on both sides, which framework has a front and a rear frame rim (2a, 2b).

4. Goggles according to Claims 1 to 3, characterised in that the frame rims (2a, 2b) on both sides of the eye glasses (3) exert such a high initial stress that the eye glasses are arranged in the frames (2) so as to be undetachably fixed and gas-tight.

5. Goggles according to Claims 1 to 4, characterised in that when the bellows (4) is in folded state, a fold part (4a) rests over a large area against the eye glass (3).

## Revendications

1. Lunettes protectrices pour la protection contre des gaz, comprenant un corps de lunettes (1) applique contre le visage de l'utilisateur, ainsi que deux espaces oculaires entourant chacun un oeil de l'utilisateur et possédant un cercle (2) pour la réception d'un verre (3), chaque espace oculaire étant réalisé comme un soufflet (4), caractérisées en ce que
   a) le soufflet (4) est formé de façon asymétrique sur la face plane du corps de lunettes (1), sous un angle $\alpha$ adéquat, et
   b) le corps de lunettes (1), les soufflets (4) et les cercles (2) pour les verres (3) sont réalisés en une seule pièce d'une matière plastique élastique possédant un haut pouvoir de reprise élastique de la forme initiale.

2. Lunettes protectrices selon la revendication 1, caractérisées en ce que la matière plastique des lunettes est de préférence un élastomere à silicone.

3. Lunettes protectrices selon les revendications 1 et 2, caractérisées en ce que les cercles (2) sont réalisés chacun comme un encadrement qui entoure le verre (3) concerné sur les deux côtés et qui possède un bord avant et un bord arrière (2a, 2b).

4. Lunettes protectrices selon les revendications 1 à 3, caractérisées en ce que les bords (2a, 2b) des cerclages exercent sur les deux côtés des verres (3) une si haute précontrainte que les verres sont maintenus de façon imperdable et étanche au gaz dans les cercles (2).

5. Lunettes protectrices selon les revendications 1 à 4, caractérisées en ce que, à l'état replié du soufflet (4), une portion de pli (4a) du soufflet s'applique contre le verre (3) par une grande surface.

Fig. 1

Fig. 2

Fig. 3

Fig. 4